# EUROPÄISCHE PATENTSCHRIFT

(11) **EP 1 184 371 B1**
(45) Veröffentlichungstag und Bekanntmachung des Hinweises auf die Patenterteilung: **29.01.2003**
(21) Anmeldenummer: 01117680.7
(22) Anmeldetag: 26.07.2001
(51) Int. Cl.: C07C 319/28

(54) **Verfahren zur Fällung von Cystin**
Process for the precipitation of cystine
Procédé de précipitation de la cystine

(30) Priorität: 17.08.2000 DE 10040177
(43) Veröffentlichungstag der Anmeldung: 06.03.2002
(73) Patentinhaber: Consortium für elektrochemische Industrie GmbH, 81379 München (DE)
(72) Erfinder: Döring, Wolfgang, Dr., 84508 Burgkirchen (DE); Stohrer, Jürgen, Dr., 82049 Pullach (DE)
(74) Vertreter: Potten, Holger

(56) Entgegenhaltungen:
- WO-A-87/05895
- DD-A1- 152 335
- DE-C- 907 175
- BARANOWSKA B. & MARCZAK E.: "Amino Acid Preparations Enriched in Cystine for Cosmetic Use. Essays of Crystallization from Pig Bristle Hydrolysates" COMUN. JORN. COM. ESP. DETERG., Bd. 21, 1990, Seiten 299-305, XP001037564

## Beschreibung

Die Erfindung betrifft ein Verfahren zur Fällung der Aminosäure Cystin aus einer schwefelsauren Lösung.

Die Aminosäure Cystin wird gewöhnlich durch saure Hydrolyse von Keratinabfällen wie z. B. menschlichen Haaren gewonnen und durch Umfällung gereinigt. Dabei ist die Umfällung aus salzsaurer Lösung gut bekannt. Mehrfach angewendet erlaubt sie die Isolierung eines kristallinen Produktes, das den Reinheitsanforderungen der Zielmärkte (Lebensmittel-Spezifikationen wie z.B. FCC IV für USA und Pharma-Spezifikationen wie z.B. USP 23 für USA, Ph.Eur.2000 für Europa) entspricht. Gewöhnlich wird die (salz-)saure Lösung des Cystins dabei zunächst auf die Fällungstemperatur gebracht und anschließend bis zu einem bestimmten pH-Wert (meistens zwischen 0,6 und 3,5) die Base, gewöhnlich wässriger Ammoniak oder wässrige Natronlauge, zudosiert (DD-0152335).

Wendet man dieses Verfahren auf eine schwefelsaure Lösung von Cystin an, so erhält man zwar ebenfalls sehr reines Cystin, welches aber immer einen gewissen Restgehalt an Sulfat enthält. Dieser beträgt, je nach Versuchsbedingungen, zwischen 400 und 1500 ppm. Der von der Ph. Eur. geforderte maximale Restgehalt an Sulfat darf jedoch nur 300 ppm betragen.

Der Erfindung lag daher die Aufgabe zugrunde, ein Verfahren bereitzustellen, welches es ermöglicht Cystin mit einem Restgehalt von weniger als 300 ppm Sulfat, welches zudem alle anderen Reinheitskriterien der gültigen Lebensmittel- und Pharma-Spezifikationen erfüllt, aus einer schwefelsauren Lösung zu fällen.

Gegenstand der Erfindung ist ein Verfahren, das dadurch gekennzeichnet ist, daß eine Mischung aus einer Lösung von Cystin in wässriger Schwefelsäure und einer wässrige Lösung einer Base durch gleichzeitige Dosierung in einen Mischbehälter gebildet wird, wobei die Dosierung derart erfolgt, daß die Mischung im Behälter einen pH-Wert zwischen 1,0 und 7,0 und eine Temperatur zwischen 30°C und dem Siedepunkt der Mischung hat.

Die Konzentration des Cystins in der zugeführten Lösung beträgt zwischen 20 g/l und 200 g/l, bevorzugt 40 g/l bis 180 g/l, besonders bevorzugt 50 bis 150 g/l.

Der molare Überschuss von Schwefelsäure in der zugeführten Lösung bezogen auf Cystin wird möglichst gering gewählt, so daß bevorzugt ein Molverhältnis H₂SO₄ : Cystin von 1,5 bis 3,0, besonders bevorzugt 2,0 bis 2,5 vorliegt.

Als wässrige Lösung einer Base kann eine wässrige Ammoniaklösung, eine wässrige Lösung eines Alkalimetallhydroxids, eines Alkalimetallhydrogencarbonates, eines Alkalimetallcarbonates oder eine Mischung dieser Lösungen eingesetzt werden.

Bevorzugt wird eine wässrige Lösung von Ammoniak, Natriumhydroxid oder Kaliumhydroxid verwendet.

Die Konzentration der Base kann in einem weiten Bereich variiert werden und beträgt bevorzugt zwischen 10 und 50 Gew.-%.

Der pH-Wert wird während der Fällung durch Einstellung der Dosierraten innerhalb eines Bereiches von 1,0 bis 7,0, bevorzugt 1,5 bis 5,0, besonders bevorzugt 1,8 bis 3,5 konstant gehalten.

Während einer einzelnen Fällung beträgt die Schwankung zwischen niedrigstem und höchstem pH-Wert in der Mischung bevorzugt weniger als 3 pH-Einheiten.

Die Temperatur während der Fällung wird zwischen 30°C und dem Siedepunkt der Mischung, bevorzugt zwischen 40°C und dem Siedepunkt der Mischung, besonders bevorzugt zwischen 50°C und dem Siedepunkt der Mischung gehalten.

Die Fällung kann sowohl in diskontinuierlicher als auch in kontinuierlicher Form durchgeführt werden.

Bei diskontinuierlicher Verfahrensführung wird zunächst eine geringe Menge an Wasser oder Mutterlauge einer vorausgegangenen Fällung im Mischbehälter vorgelegt. Nachdem der Mischbehälterinhalt auf Fällungstemperatur gebracht wurde, werden anschließend die Komponenten wie beschrieben zudosiert. Eine besonders langsame Dosierung wirkt sich dabei generell positiv auf die Reinheit des Produktes aus, aus wirtschaftlichen Gründen ist die Gesamtdauer der Dosierung dennoch vorzugsweise zwischen 0,5 und 6 h, besonders bevorzugt zwischen 1 und 3 h zu wählen.

Die Komponenten können wahlweise vor der Einbringung in den Mischbehälter einzeln auf die Fällungstemperatur aufgeheizt werden oder erst im Mischbehälter auf die gewünschte Fällungstemperatur gebracht werden.

Bei kontinuierlicher Fahrweise werden die Komponenten vor der Einbringung in den Mischbehälter einzeln auf die Fällungstemperatur aufgeheizt und anschließend in den Mischer dosiert.

Nach der durchgeführten Fällung wird die Mischung durch eine Fest-Flüssig-Trennung wie z. B. Zentrifugation, Filtration oder Sedimentation in Kristallisat und Mutterlauge getrennt.

Bevorzugte Fest-Flüssig-Trennungen sind Zentrifugation und Filtration.

Die Abtrennung des Kristallisats von der Mutterlauge kann durchgeführt werden wie für die Abtrennung nach Fällung aus salzsauerer Lösung bekannt, wobei vorteilhafterweise im erfindungsgemäßen Verfahren keine Korrosionsprobleme auftreten, wie mit den Chlorid-haltigen, sauren Lösungen aus dem Stand der Technik.

Anschließend erfolgt ein Nachwaschen des Kristallisates mit Wasser, bevorzugt mit entionisiertem Wasser. Die Wassermenge wird so bemessen, daß im ablaufenden Filtrat mit 10%iger Bariumchloridlösung kein Sulfat mehr nachgewiesen werden kann. Bevorzugt werden 5 bis 20 Volumina Wasser bezogen auf Trockengewicht Kristallisat eingesetzt. Die Wassermenge wird dabei bevorzugt auf drei bis 20 Portionen aufgeteilt.

Die folgenden Beispiele dienen der weiteren Erläuterung der Erfindung.

### Beispiel 1

In einem 1-1-Glasrührreaktor wurden 250 ml H₂O vorgelegt. Nachdem auf 60°C aufgeheizt wurde, wurden bei dieser Temperatur innerhalb von 2 h unter Rühren 340 ml einer schwefelsauren Cystin-Lösung mit einem Gehalt von ca. 100 g/l L-Cystin zudosiert. Gleichzeitig wurde eine 25 Gew.-%ige wässrige Ammoniaklösung so zudosiert, daß ein pH-Wert von ca. 3,0 gehalten wurde. Anschließend wurde auf 20 °C abgekühlt, der ausgefallene Feststoff abfiltriert und mit insgesamt 600 ml H₂O gewaschen. Nach Trocknung im Vakuum bei 70°C erhielt man 33,3 g (98%) L-Cystin. Der Sulfatgehalt des Produktes betrug 275 ppm.

### Beispiel 2

In einem 1-1-Glasrührreaktor wurden 250 ml H₂O vorgelegt. Nachdem auf 80°C aufgeheizt wurde, wurden bei dieser Temperatur innerhalb von 2 h unter Rühren 400 ml einer schwefelsauren Cystin-Lösung mit einem Gehalt von ca. 93 g/l L-Cystin zudosiert. Gleichzeitig wurde eine 25 Gew.-%ige wässrige Ammoniaklösung so zudosiert, daß ein pH-Wert von ca. 2,3 gehalten wurde. Anschließend wurde auf 20 °C abgekühlt, der ausgefallene Feststoff abfiltriert und mit insgesamt 600 ml H₂O gewaschen. Nach Trocknung im Vakuum bei 70°C erhielt man 36,0 g (97%) L-Cystin. Der Sulfatgehalt des Produktes betrug 208 ppm.

### Beispiel 3

In einem 1-1-Glasrührreaktor wurden 250 ml H₂O vorgelegt.

Nachdem auf 60°C aufgeheizt wurde, wurden bei dieser Temperatur innerhalb von 2 h unter Rühren 360 ml einer schwefelsauren Cystin-Lösung mit einem Gehalt von ca. 100 g/l L-Cystin zudosiert. Gleichzeitig wurde eine 20 Gew.-%ige wässrige Natriumhydroxidlösung so zudosiert, daß ein pH-Wert von ca. 4,0 gehalten wurde. Anschließend wurde auf 20 °C abgekühlt, der ausgefallene Feststoff abfiltriert und mit insgesamt 600 ml H₂O gewaschen. Nach Trocknung im Vakuum bei 70°C erhielt man 34,7 g (97%) L-Cystin. Der Sulfatgehalt des Produktes betrug 170 ppm.

### Beispiel 4

In einem 60-1-Emaillekessel wurden 15 1 H₂O vorgelegt und auf 60°C erhitzt. Unter Rühren wurden bei dieser Temperatur innerhalb von 2 h 46 1 einer schwefelsauren Cystin-Lösung mit einem Gehalt von ca. 82 g/l L-Cystin zudosiert. Gleichzeitig wurde eine 25 Gew.-%ige wässrige Ammoniaklösung so zudosiert, daß ein pH-Wert von ca. 3,0 gehalten wurde. Anschließend wurde auf 20 °C abgekühlt, der ausgefallene Feststoff abfiltriert und mit 10 Portionen von je 10 1 H₂O gewaschen. Nach Trocknung im Vakuum bei 70°C erhielt man 3,57 kg (95%) L-Cystin. Der Sulfatgehalt des Produktes betrug 290 ppm.

### Vergleichsbeispiel 1

In einem 1-1-Glasrührreaktor wurden 250 ml H₂O vorgelegt. Bei 20°C wurden innerhalb von 2 h unter Rühren 360 ml einer schwefelsauren Cystin-Lösung mit einem Gehalt von ca. 100 g/l L-Cystin zudosiert. Gleichzeitig wurde eine 25 Gew.-%ige wässrige Ammoniaklösung so zudosiert, daß ein pH-Wert von ca. 3,0 gehalten wurde. Anschließend wurde der ausgefallene Feststoff abfiltriert und mit insgesamt 600 ml H₂O gewaschen. Nach Trocknung im Vakuum bei 70°C erhielt man 35,2 g (98%) L-Cystin. Der Sulfatgehalt des Produktes betrug 580 ppm.

### Vergleichsbeispiel 2

In einem 100-1-Emaillekessel wurden 74 1 einer schwefelsauren Cystin-Lösung mit einem Gehalt von ca. 104 g/l L-Cystin vorgelegt und auf 60°C aufgeheizt. Anschließend wurden 7,0 kg einer 25 Gew.-%igen wässrigen Ammoniaklösung bei dieser Temperatur innerhalb von 40 min zugegeben. Der pH-Wert nach der Zugabe betrug 3,0. Nach Abkühlen auf 20°C wurde filtriert, mit 12 Portionen von je 10 1 H₂O gewaschen und im Vakuum bei 70°C getrocknet. Man erhielt 7,43 kg (97%) L-Cystin. Der Sulfatgehalt des Produktes betrug 800 ppm.

## Patentansprüche

1. Verfahren zur Fällung von Cystin aus einer schwefelsauren Lösung, **dadurch gekennzeichnet, daß** eine Mischung aus einer Lösung von Cystin in wässriger Schwefelsäure und einer wässrigen Lösung einer Base durch gleichzeitige Dosierung in einem Mischbehälter gebildet wird, wobei die Dosierung derart erfolgt, daß die Mischung im Behälter einen pH-Wert zwischen 1,0 und 7,0 und eine Temperatur zwischen 30°C und dem Siedepunkt der Mischung hat.

2. Verfahren nach Anspruch 1, **dadurch gekennzeichnet, daß** die Konzentration des Cystins in der wässrigen Schwefelsäure zwischen 20 und 200 g/l beträgt.

3. Verfahren nach einem der Ansprüche 1 oder 2, **dadurch gekennzeichnet, daß** die Lösung von Cystin in wässriger Schwefelsäure ein Molverhältnis H₂SO₄ : Cystin von 1,5 bis 3,0 besitzt.

4. Verfahren nach einem der Ansprüche 1 bis 3, **dadurch gekennzeichnet, daß** als wässrige Lösung einer Base eine wässrige Ammoniaklösung, eine wässrige Lösung eines Alkalimetallhydroxids, eines Alkalimetallhydrogencarbonates, eines Alkalimetallcarbonates oder eine Mischung dieser Lösungen eingesetzt werden.

5. Verfahren nach Anspruch 4, **dadurch gekennzeichnet, daß** als wässrige Lösung einer Base eine wässrige Lösung von Ammoniak, Natriumhydroxid oder Kaliumhydroxid eingesetzt wird.

6. Verfahren nach einem der Ansprüche 1 bis 5, **dadurch gekennzeichnet, daß** die Konzentration der Base zwischen 10 und 50 Gew.-% beträgt.

7. Verfahren nach einem der Ansprüche 1 bis 5, **dadurch gekennzeichnet, daß** während einer Fällung die Schwankung zwischen niedrigstem und höchstem pH-Wert in der Mischung bevorzugt weniger als 3 pH-Einheiten beträgt.

8. Verfahren nach einem der Ansprüche 1 bis 7, **dadurch gekennzeichnet, daß** nach der Fällung durch eine Fest-Flüssig-Trennung ein Kristallisat abgetrennt wird.

9. Verfahren nach Anspruch 8, **dadurch gekennzeichnet, daß** als Fest-Flüssig-Trennung eine Filtration oder eine Zentrifugation verwendet wird.

10. Verfahren nach Anspruch 8 oder 9, **dadurch gekennzeichnet, daß** das Kristallisat mit Wasser nachgewaschen wird, wobei die Wassermenge so bemessen wird, daß im ablaufenden Filtrat mit 10%iger Bariumchloridlösung kein Sulfat mehr nachgewiesen werden kann.

## Claims

1. Process for precipitating cystine from a solution in sulphuric acid, **characterized in that** a mixture of a solution of cystine in aqueous sulphuric acid and of an aqueous solution of a base is formed by simultaneous metering in a mixing container, the metering taking place in such a way that the mixture in the container has a pH between 1.0 and 7.0 and a temperature between 30°C and the boiling point of the mixture.

2. Process according to Claim 1, **characterized in that** the cystine concentration in the aqueous sulphuric acid is between 20 and 200 g/l.

3. Process according to either of Claims 1 or 2, **characterized in that** the solution of cystine in aqueous sulphuric acid has an H₂SO₄:cystine molar ratio of from 1.5 to 3.0.

4. Process according to any of Claims 1 to 3, **characterized in that** an aqueous ammonia solution, an aqueous solution of an alkali metal hydroxide, of an alkali metal bicarbonate, of an alkali metal carbonate or a mixture of these solutions is employed as aqueous solution of a base.

5. Process according to Claim 4, **characterized in that** an aqueous solution of ammonia, sodium hydroxide or potassium hydroxide is employed as aqueous solution of a base.

6. Process according to any of Claims 1 to 5, **characterized in that** the concentration of the base is between 10 and 50% by weight.

7. Process according to any of Claims 1 to 5, **characterized in that** the variation between the lowest and highest pH in the mixture during a precipitation is preferably less than 3 pH units.

8. Process according to any of Claims 1 to 7, **characterized in that** crystals are removed by a solid/liquid separation after the precipitation.

9. Process according to Claim 8, **characterized in that** a filtration or centrifugation is used as solid/liquid separation.

10. Process according to Claim 8 or 9, **characterized in that** the crystals are washed with water, with the amount of water being such that sulphate is no longer detectable with 10% strength barium chloride solution in the filtrate which drains off.

## Revendications

1. Procédé pour la précipitation de cystine à partir d'une solution acidifiée à l'acide sulfurique, **caractérisé en ce qu'**on prépare dans un récipient de mélange un mélange constitué d'une solution de cystine dans de l'acide sulfurique aqueux et d'une solution aqueuse d'une base, par addition réglée simultanée, en effectuant l'addition réglée de manière que le mélange dans le récipient ait un pH entre 1,0 et 7,0 et une température entre 30°C et le point d'ébullition du mélange.

2. Procédé selon la revendication 1, **caractérisé en ce que** la concentration de la cystine dans l'acide sulfurique aqueux est comprise entre 20 et 200 g/l.

3. Procédé selon la revendication 1 ou 2, **caractérisé en ce que** la solution de cystine dans l'acide sulfurique aqueux présente un rapport molaire H₂SO₄:cystine de 1,5 à 3,0.

4. Procédé selon l'une quelconque des revendications 1 à 3, **caractérisé en ce qu'**on utilise comme solution aqueuse d'une base une solution aqueuse d'ammoniac, une solution aqueuse d'un hydroxyde de métal alcalin, une solution aqueuse d'un hydrogénate de métal alcalin, d'un carbonate de métal alcalin ou un mélange de ces solutions.

5. Procédé selon la revendication 4, **caractérisé en ce qu'**on utilise comme solution aqueuse d'une base une solution aqueuse d'ammoniac, d'hydroxyde de sodium ou d'hydroxyde de potassium.

6. Procédé selon l'une quelconque des revendications 1 à 5, **caractérisé en ce que** la concentration de la base est comprise entre 10 et 50 % en poids.

7. Procédé selon l'une quelconque des revendications 1 à 5, **caractérisé en ce que**, pendant une précipitation, la variation entre le pH le plus bas et le pH le plus élevé dans le mélange est de préférence inférieure à 3 unités de pH.

8. Procédé selon l'une quelconque des revendications 1 à 7, **caractérisé en ce que**, après la précipitation, on sépare un cristallisat par une séparation liquide-solide.

9. Procédé selon la revendication 8, **caractérisé en ce que**, en tant que séparation liquide-solide, on utilise une filtration ou une centrifugation.

10. Procédé selon la revendication 8 ou 9, **caractérisé en ce que** le cristallisat est ensuite lavé avec de l'eau, la quantité d'eau étant choisie de manière à ne pouvoir plus détecter de sulfate dans le filtrat s'écoulant, à l'aide d'une solution de chlorure de baryum à 10 %.
